# EUROPÄISCHE PATENTSCHRIFT

(11) **EP 0 705 609 B1**
(45) Veröffentlichungstag und Bekanntmachung des Hinweises auf die Patenterteilung: **05.04.2000**
(21) Anmeldenummer: 95114690.1
(22) Anmeldetag: 19.09.1995
(51) Int. Cl.: A61L 24/00, A61L 27/00

(54) **Poröse Knochenersatzmaterialien**
Porous bone-replacing material
Matériau poreux de remplacement pour les os

(30) Priorität: 06.10.1994 DE 4435680
(43) Veröffentlichungstag der Anmeldung: 10.04.1996
(73) Patentinhaber: MERCK PATENT GmbH, 64293 Darmstadt (DE)
(72) Erfinder: Nies, Berthold, Dr., D-64407 Fränkisch-Crumbach (DE); Tröster, Sabine, Dr., D-63225 Langen (DE); Specht, Rainer, Dr., D-90765 Fürth (DE)

(56) Entgegenhaltungen:
- EP-A- 0 361 408
- WO-A-86/02370
- DD-A- 207 655
- FR-A- 2 606 403
- US-A- 4 373 217
- US-A- 4 842 603
- US-A- 5 338 772

## Beschreibung

Die Erfindung betrifft poröse Knochenersatzmaterialien und insbesondere ein Verfahren zu deren Herstellung.

Knochenzemente auf Basis von Acrylatkunststoffen sowie daraus hergestellte Knochenersatzmaterialien sind seit langem bekannt. Polymermaterialien auf Basis von Acryl- und/oder Methacrylsäureestern haben sich hier aufgrund ihrer Biokompatibilität, ihrer vorzüglichen Festigkeitseigenschaften, ihrer günstigen Eigenschaften bei der Freisetzung eingelagerter pharmazeutischer Wirkstoffe und nicht zuletzt aufgrund ihrer anwendungsgerechten Verarbeitbarkeit bewährt.

Gängige Knochenzemente setzen sich aus einer Feststoffkomponente, die aus einem feinteiligen Polymerisat von Acryl- und/oder Methacrylsäureestern und weiteren Zusätzen wie Polymerisationskatalysatoren sowie gegebenenfalls Röntgenkontrastmitteln, Füllstoffen und Farbstoffen besteht, und aus einer flüssigen Komponente, die aus einem Acryl- und/ oder Methacrylsäureestermonomeren und weiteren Zusätzen wie Polymerisationsbeschleunigern und Stabilisatoren besteht, zusammen. Zum Gebrauch werden Feststoffkomponente und flüssige Komponente zu einer flüssigen bis halbfesten Paste vermengt, diese gegebenenfalls in eine gewünschte Form gebracht oder zur Einzementierung einer Prothese an dem Implantationsort appliziert. Die Aushärtung der Masse erfolgt durch die mit dem Vermischen der Komponenten induzierte Polymerisationsreaktion. Der Knochenzement wird zweckmäßigerweise in einer solchen Form bereitgestellt, daß getrennte Behältnisse mit aufeinander abgestimmten Mengen der beiden Komponenten als Packungseinheit zusammengefaßt sind. In aller Regel beträgt der Anteil an Feststoffkomponente etwa 50 bis 75 Gew.% und der Anteil der flüssigen Komponente etwa 50 bis 25 Gew.%.

Sehr gebräuchlich ist zum Beispiel ein Knochenzement, der in einer Normalpackung 2 Beutel mit je etwa 40 g Polymerpulver und 2 Ampullen mit je 20 ml Monomerflüssigkeit enthält. Das Pulver ist ein feines Perlpolymerisat aus Methacrylsäuremethylester mit einem Copolymeranteil von Acrylsäuremethylester. Als Katalysator sind dem Pulver etwa 0,5 % Dibenzoylperoxid zugesetzt. Zur Kennzeichnung des Materials sind bei der Herstellung geringe Mengen von Chlorophyll miteinpolymerisiert. Das Pulver enthält zusätzlich ein übliches Röntgenkontrastmittel wie zum Beispiel Zirkondioxid. Die zugehörige Flüssigkeit besteht aus monomerem Methacrylsäuremethylester, dem als Polymerisationsbeschleuniger etwa 0,7 % Dimethyl-p-toluidin sowie als Stabilisator geringe Mengen von Hydrochinon zugesetzt sind. Auch diese Flüssigkeit ist in der Regel zur Kennzeichnung mit einer geringen Menge von Chlorophyll eingefärbt. Das in Polyethylenbeutel abgepackte Pulver ist mit Ethylenoxid sterilisiert. Die Flüssigkeit ist sterilfiltriert und in Glasampullen abgefüllt.

Beim Zusammenmischen von 2 Gewichtsteilen Pulver mit einem Gewichtsteil Flüssigkeit reagiert das Dibenzoylperoxid mit dem Dimethyl-p-toluidin in der Flüssigkeit, wodurch die radikalische Polymerisation angeregt wird. Die Mischung ist so abgestimmt, daß sie schon nach etwa einer Minute als teigige Paste verwendet werden kann. Diese Paste bleibt für mehrere Minuten knetbar und beginnt dann unter Wärmeentwicklung auszuhärten. Nach etwa 5 bis 10 Minuten ist die Polymerisation im wesentlichen abgeschlossen. Während der Polymerisationsphase, solange die Paste noch formbar ist, kann diese in jede gewünschte Form gebracht werden, also zum Beispiel zum Ausfüllen von Knochenhöhlen oder zum Einzementieren von Prothesen direkt in den Körper gebracht oder zur Herstellung von Formkörpern verwendet werden, die extrakorporal aushärten und danach an beliebigen Körperstellen eingesetzt werden können.

Während die klinischen Ergebnisse mit derartigen Knochenzementen bei der Implantation von Endoprothesen, wobei die Prothese in aller Regel lediglich mit einem gleichmäßigen dünnen Zementmantel umgeben ist, der die Verbindung zwischen Prothese und Knochenbett herstellt, überwiegend sehr gut sind, stellen sich Probleme bis hin zum klinischen Versagen häufig dann ein, wenn aufgrund der Implantationsbedingungen oder des Einsatzgebietes relativ große und dicklagige Zementmengen erforderlich sind. Dies ist zum Beispiel dann der Fall, wenn bei einem Prothesenwechsel oder nach der Resektion von Knochentumoren relativ große Knochendefekte vorhanden sind, die mit Knochenzement aufgefüllt werden müssen. Ein Grund für die auftretenden Probleme liegt in der exothermen Polymerisationsreaktion bei der Aushärtung des Knochenzementes. Bei Zementdicken ab etwa 4 mm treten deutliche Temperaturerhöhungen auf, da die entstehende Reaktionswärme nicht mehr ausreichend verteilt und abgeführt werden kann. Beispielsweise kann im Inneren eines zylindrischen Knochenzementformkörpers von etwa 3 cm Durchmesser bei der Aushärtung ohne weiteres eine Temperatur von etwa 100 °C erreicht werden. Hitzenekrosen im Knochenlager oder im den Implantationsort umgebenden Gewebe sind die Folge.

Ein weiterer Problemfaktor ist die Schwindung von Knochenzement auf Acrylatbasis, die um so mehr ins Gewicht fällt, je dicker die Zementschicht ist. Dies verursacht Schädigungen des Implantatlagers, was zu vorzeitiger Lockerung bis hin zum Ausbrechen der Prothese führen kann.

Bei der Implantation von Endoprothesen wie auch bei implantierbaren Formkörpern für den Knochenersatz im Rahmen der Osteosynthese ist ein möglichst fester Verbund mit dem ursprünglichen Knochen bzw. seinen Fragmenten anzustreben. Dies läßt sich effektiv nur bei einer innigen Verzahnung bis idealerweise hin zur völligen Durchbauung des Implantatwerkstoffes durch neugebildete Knochenmatrix erreichen. Voraussetzung hierfür ist allerdings eine ausreichende Porosität, idealerweise mit interkonnektierendem Porensystem, des Knochenersatzmaterials.

Knochenersatzwerkstoffe mit poröser, gegebenenfalls auch interkonnektierender Porenstruktur bei gleichzeitig hoher mechanische Stabilität sind bekannt. Es handelt sich hierbei im wesentlichen jedoch um keramische Formkörper, die durch Sintern von beispielsweise Calciumphosphatmaterialien wie Hydroxylapatit oder Tricalciumphosphat oder durch Pyrolyse und Sintern von natürlichen Knochen erhalten werden. Naturgemäß können mit diesen Materialien nur Knochendefekte aufgefüllt werden.

Aus EP 0 519 293 A1 ist ein poröses Implantatmaterial mit interkonnektierendem Porensystem auf Basis von Calciumphosphatkeramikpartikeln und bioresorbierbarem Polymer bekannt. Dieses Material eignet sich ebenfalls nur zur Auffüllung von Knochendefekten und ist aufgrund seiner geringen mechanischen Festigkeit nicht für den Ersatz hochbelasteter Knochenstrukturen geeignet. Auch wenn dieses Material in gewissem Ausmaß plastisch verformbar ist, so eignet es sich nicht im Sinne von Knochenzement für die Verankerung von Endoprothesen.

Bei üblichen Knochenzementen wird aus Gründen der angestrebten mechanischen Festigkeit des Verbundes Prothese-Knochenzement-Knochenbett eine möglichst geringe Porosität angestrebt. Aus diesem Grund erfolgt bevorzugt die Anmischung der Knochenzementkomponenten im Vakuum und mit anschließender Kompression, um hierdurch Lufteinschlüsse und daraus resultierende Porenbildung möglichst zu vermeiden. Um den Langzeitverbund mit dem Knochenlager zu verbessern, ist es vorteilhaft dem Knochenzement osteokonduktive Zusätze zuzufügen. Als solche Zusätze kommen vornehmlich feinteilige Calciumphosphatmaterialien wie Hydroxylapatit und Tricalciumphosphat, die mehr oder weniger bioresorbierbar sind, in Betracht. Aus EP 0 016 906 und EP 0 148 253 sind solche Knochenzemente bekannt, die bis zu 35 Gew.% an derartigen Calciumphosphaten mit Partikelgröße bis zu 300 µm enthalten können. Die Partikel sind jedoch größtenteils im Polymermaterial des Knochenzementes eingebettet und von diesem umhüllt. Eine gewisse Porosität, in die Knochenmatrix einwachsen kann, kann sich daher nur im Laufe der Einheilung der zementierten Prothese bzw. des Knochenzementimplantates in den im Kontakt mit dem Knochenbett stehenden Oberflächenbereichen des Knochenzementes durch Resorption oberflächiger Calciumphosphatpartikel ausbilden.

Der Erfindung lag die Aufgabe zugrunde ein Knochenersatzmaterial aufzufinden, das gleichermaßen als Knochenzement für die Verankerung von Endoprothesen wie auch zur Herstellung von Implantatformkörpern geeignet ist und das eine Porosität mit möglichst interkonnektierendem Porensystem bei ausreichender mechanischer Festigkeit aufweist.

Es wurde nun gefunden, daß sich ein derartiges Knochenersatzmaterial erhalten läßt, wenn man
(a) 0 bis 48 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen
(b) 2 bis 50 Gew.% einer flüssigen Komponente, bestehend aus Acryl- und/oder Methacrylsäureestermonomeren sowie gegebenenfalls weiteren Zusätzen wie Polymerisationsbeschleunigern und Stabilisatoren und
(c) 50 bis 98 Gew.% eines grobteiligen Granulates, aus einem biokompatiblen Material mit größtem Partikeldurchmesser von 0,5 bis 10 mm
miteinander vermengt, gegebenenfalls in eine gewünschte Form bringt und dann aushärtet. Man erhält hierbei ein Knochenersatzmaterial mit teilweise oder vollständig interkonnektierendem Porensystem mit einem Volumenanteil von 5 bis 60 %.

Gegenstand der Erfindung ist somit ein wie vorstehend charakterisiertes Verfahren zur Herstellung von porösen Knochenersatzmaterialien, die ein teilweise oder vollständig interkonnektierendes Porensystem mit einem Volumenanteil von 5 bis 60 % aufweisen.

Bei dem erfindungsgemäßen Verfahren lassen sich alle üblichen Knochenzemente auf Acrylat-/Methacrylat-Basis bzw. die hierfür gebräuchlichen Ausgangsprodukte verwenden. Knochenzemente dieser Art sind im Handel erhältlich. Ihre Zusammensetzung und die Art ihrer Verarbeitung sind dem Fachmann geläufig.

Erfindungsgemäß bildet ein grobteiliges Granulat aus einem biokompatiblen Material mit größtem Partikeldurchmesser von 0,5 bis 10 mm einen wesentlichen Anteil, nämlich 50 bis 98 Gew.%, bezogen auf die Gesamtmenge, an dem porösen Knochenersatzmaterial. Vorzugsweise liegt der größte Partikeldurchmesser des Granulates zwischen 1 und 5 mm und insbesondere um etwa 3 mm. Als "größter Partikeldurchmesser" ist bei nichtkugelförmigen bzw. unregelmäßig geformten Teilchen die längste durch das Teilchen legbare Achse zu verstehen. Die Form- und Größenverteilung der Granulatpartikel kann im Prinzip nach Belieben gewählt werden. Neben im wesentlichen unregelmäßig geformten Partikeln sind bevorzugt Granulatpartikel mit Kugelform, angenäherter Kugelform und insbesondere mit Zylinderform. Vorzugsweise liegt die Partikelgröße in einem engen Bereich oder ist weitestgehend einheitlich. Die Auswahl von Form und Größe der Partikel ergibt sich nach der in dem Knochenersatzmaterial angestrebten Porosität und Art der Porenstruktur. So führen beispielsweise kugelförmige Partikel zu insgesamt dichteren Materialien mit geometrisch gleichmäßigerer Porencharakteristik, während etwa zylinderförmige oder völlig unregelmäßige Partikel zu poröseren Materialien mit unregelmäßigerer Porenstruktur führen. Weiterhin führen grobe Partikel zu größeren Porendurchmessern, während feine Partikel wiederum ein engeres Porensystem bewirken.

Als Ausgangsmaterialien für das grobteilige Granulat kommen im Prinzip alle biokompatiblen Kunststoffe und biokompatiblen anorganischen Feststoffe in Betracht. Vorzugsweise werden solche Materialien eingesetzt, die in der Endoprothetik gebräuchlich und bewährt sind. Besonders zweckmäßig sind Materialien, die einen innigen, festen Verbund mit den Komponenten von Knochenzementen, insbesondere mit dem aushärtenden Monomer, eingehen. Bevorzugte Materialien basieren demgemäß auf Polyacrylaten und/oder Polymethacrylaten. Polymere dieser Art stehen in Granulatform im gewünschten Größenbereich der Granulatpartikel zur Verfügung oder können ohne weiteres zu entsprechenden Granulaten, etwa durch Extrusion und Zerkleinerung verarbeitet werden. Besonders günstig ist es, wenn als Granulatmaterial das Knochenzementbasismaterial selbst eingesetzt wird. Demgemäß besteht dann ein solches Granulat aus einem ausgehärteten Gemisch von etwa 5 bis 90 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, und von etwa 95 bis 10 Gew.% einer flüssigen Komponente, bestehend aus einem Acryl- und/oder Methacrylsäureestermonomeren sowie gegebenenfalls weiteren Zusätzen wie Polymerisationsbeschleunigern und Stabilisatoren. Eine derartige Knochenzementmischung kann nach erfolgter Aushärtung zur gewünschten Partikelgröße mechanisch zerkleinert werden. Hierbei resultieren in aller Regel Granulatpartikel mit unregelmäßiger Formgebung. Der frisch angemischte Knochenzement kann auch während der flüssigen bzw. plastischen Phase zu Granulatpartikeln ausgeformt werden, beispielsweise durch Extrusion. Hierbei resultieren in aller Regel zylinderförmige Granulatpartikel. Eine elegante Methode zur Herstellung kugelförmiger Knochenzementpartikeln ist etwa die, einen frisch angemischten niedrigviskosen Knochenzement in eine gerührte und auf Reaktionstemperatur temperierte wäßrige Natriumalginatlösung zu tropfen. Hierdurch lassen sich Polymerisatperlen von etwa 0,5 bis 3 mm Durchmesser erzeugen, je nach Düsengröße, Viskosität des Knochenzementes und Rührgeschwindigkeit der Alginatlösung.

Neben Materialien auf Acrylat-/Methacrylat-Basis können auch andere Kunststoffmaterialien wie Polyolefine, Copolymere von Acrylaten mit Styrol und/oder Butadien sowie Epoxidharze für die Granulatherstellung verwendet werden.

Von anorganischen Materialien sind Calciumverbindungen wie insbesondere Calciumphosphate bevorzugt. Besonders bevorzugt liegen diese in Form von gesinterter Calciumphosphatkeramik vor. Ausgangsstoffe zur Herstellung von Granulaten nach an sich bekannten Methoden können sein Hydroxylapatit, Tricalciumphosphat oder pyrolisierter und zur Keramik gesinterter Knochen.

Die Feststoffkomponente des Knochenzementes, die üblicherweise als Perlpolymerisat von Methylmethacrylat-Methylacrylat-Copolymer mit Partikelgrößen zwischen 5 und 250 µm vorliegt, enthält einen Polymerisationskatalysator wie etwa Dibenzoylperoxid. Weiterhin kann sie Röntgenkontrastmittel wie zum Beispiel Zirkondioxid, Farbstoffe zur Kennzeichnung wie etwa Chlorophyll sowie Füllstoffe und gegebenenfalls weitere Zusätze enthalten. Die flüssige Monomerkomponente Methylmethacrylat enthält in aller Regel einen Polymerisationsbeschleuniger wie Dimethyl-p-toluidin sowie Hydrochinon als Stabilisator in den hierfür üblichen Mengen. Als flüssige Komponente kommen auch Lösungen oder Suspensionen von Oligomeren und/oder Polymeren von Acrylaten und/oder Methacrylaten in den genannten Monomeren in Betracht. Weiterhin können Farbstoffe und sonstige zweckmäßige Zusätze vorhanden sein. Als Zusätze zur Feststoffkomponente wie auch für das grobteilige Granulat kommen insbesondere osteoinduktive und/oder osteokonduktive Füllstoffe wie beispielsweise Hydroxylapatit und Tricalciumphosphat in Frage. Der Anteil derartiger Zusätze kann in einem weiten Bereich variieren und ist abhängig vom jeweiligen Anforderungsprofil des Knochenzementes bzw. der entsprechenden Folgeprodukte. Er übersteigt in der Regel kaum 30 Gew.%., bezogen auf die Feststoffkomponente bzw. auf das grobteilige Granulat.

Dem erfindungsgemäßen Knochenzement bzw. seinen Komponenten können weiterhin alle pharmazeutischen Wirkstoffe zugesetzt werden, die zum einen von ihrem Wirkungsprofil her in Knochenzementen, in Knochenersatzwerkstoffen sowie in implantierbaren Pharmakadepots sinnvoll sind und die zum anderen gegenüber den Bestandteilen von Knochenzementen sowie den bei der Aushärtung entstehenden Temperaturen ausreichend stabil sind. Als Wirkstoffe kommen vorzugsweise Cytostatika wie Methotrexat, Cisplatin, Cyclophosphamid, Fluoruracil, Doxorubicin etc., Antibiotika wie Gentamicin, Clindamycin, Vancomycin, Teicoplanin etc., weiterhin Antiseptika sowie knochenwachstumsfördernde Substanzen in Betracht. In aller Regel ist ein Anteil an pharmazeutischem Wirkstoff von 0,1 bis 5 Gew.%, bezogen auf die Gesamtmenge an Knochenzement, ausreichend; in Einzelfällen, insbesondere bei der Herstellung von implantierbaren Pharmakadepots, kann der Wirkstoffanteil auch höher liegen.

Das poröse Knochenersatzmaterial wird zu seiner Herstellung zweckmäßigerweise in Form eines Sets bereitgestellt, das sich aus getrennten Packungen der drei Hauptkomponenten zusammensetzt. Komponente (a) beinhaltet die Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, deren Anteil etwa 0 bis 48 Gew.% des Knochenzementes beträgt. Komponente (b) beinhaltet die flüssige Komponente, bestehend aus Acryl- und/oder Methacrylsäureestermonomeren sowie gegebenenfalls weiteren Zusätzen wie Polymerisationsbeschleunigern und Stabilisatoren, deren Anteil etwa 2 bis 50 Gew.% des Knochenersatzmaterials beträgt. Komponente (c) beinhaltet das grobteilige Granulat aus biokompatiblen Material mit größtem Partikeldurchmesser von 0,5 bis 10 mm, dessen Anteil etwa 50 bis 98 Gew.%, bezogen auf das Knochenersatzmaterial, beträgt.

Vorzugsweise sind die Mengen der Komponenten so aufeinander abgestimmt, daß die gesamten drei Packungsinhalte miteinander vereinigt werden. Die Mengenabstimmung erfolgt nach Maßgabe des vorgesehenen Anwendungszweckes und je nach dem, ob eine niedrigviskose, eine mittelviskose oder eine hochviskose Mischung erwünscht ist. Erforderlichenfalls ist die Feststoffkomponente und das Granulat einer Endsterilisation mittels Strahlung oder Ethylenoxid und die flüssige Monomerkomponente einer Sterilfiltration unterzogen worden und jeweils steril in geeignete Packmittel abgefüllt. Die Feststoffkomponente (a) und das grobteilige Granulat (c) können gegebenenfalls auch als Gemisch in einer Packungseinheit vorliegen.

Zweckmäßig ist die Ergänzung dieses Sets mit einer Vorrichtung zum Anmischen und/oder Ausbringen des Knochenzementes. Entsprechende Vorrichtungen sind bekannt und gebräuchlich. Vorzugsweise ermöglichen entsprechende Vorrichtungen das Anmischen des Knochenzementes unter Vakuum sowie das kombinierte Ausbringen des Zementes mittels einer Knochenzementspritze.

Die Herstellung des gebrauchsfertigen porösen Knochenzementes bzw. Knochenersatzmaterials und seine weitere Verarbeitung erfolgen in völliger Analogie zu bisherigen Knochenzementsystemen. Die drei Hauptkomponenten werden zusammengebracht und miteinander vermischt. Nach inniger Durchmischung der Komponenten setzt durch den enthaltenen Katalysator die Polymerisation ein; für den Zeitraum einiger Minuten bleibt die Masse flüssig bis plastisch verformbar; danach liegt das ausgehärtete Endprodukt vor.

Je nach den mengenmäßigen Anteilen der drei Hauptkomponenten sowie der Partikelform und Partikelgröße der Granulatkomponente wird hierbei ein feinporöses bis grobporiges Material erhalten, wobei der Volumenanteil der Poren von 5 bis 60 % reichen kann.

Charakteristisch hierbei ist die Ausbildung eines teilweisen bis vollständigen interkonnektierenden Porensystems. Überraschend zeigt sich, daß bei der Aushärtung selbst großvolumiger Massen nur ein geringfügiger Temperaturanstieg festzustellen ist. Bei der Applikation im belebten Organismus sind daher Hitzenekrosen auszuschließen. Im ausgehärteten Zustand weist das poröse Knochenersatzmaterial eine vorzügliche mechanische Stabilität wie insbesondere eine hohe Druckfestigkeit auf.

Das poröse Knochenersatzmaterial kann in üblicher Weise während des flüssigen bzw. plastischen Stadiums als Knochenzement für die Implantation von Knochenprothesen verwendet werden. Der Chirurg kann auch die Masse zu Formkörpern beliebiger Form und Größe verarbeiten und diese nach der Aushärtung zur Rekonstitution von Knochendefekten oder als lokale Wirkstoffdepots in die zu behandelnden Körperbereiche implantieren. Derartige implantierbare Formkörper oder Pharmakadepots können auch bereits vorgefertigt angeboten werden.

### Beispiel 1:

Niedrig viskoser Knochenzement der Zusammensetzung 31 g Polymethylmethacrylat/Polymethylacrylat (94/6)-Copolymer, 6 g Hydroxylapatit-Pulver, 3 g Zirkondioxid wird mit 30 ml Methylmethacrylat-Monomer auf übliche Weise angerührt. Die Komponenten enthalten das Startersystem Dibenzoylperoxid/Dimethyl-p-toluidin. Zu diesem Teig werden 100 g reines, zylinderförmiges Polymethylmethacrylat-Granulat (Durchmesser 2 mm, Länge 3 mm) zugegeben und mit dem Knochenzement-Teig gründlich gemischt. Die gemischte Masse wird in Polypropylen-Formen gegeben (Durchmesser 30 mm, Höhe 10 mm) und härtet nach einer Dauer von ca. 15 Min. aus. Es resultiert ein interkonnektierend poröser Körper mit einer Porosität von 20 %. Die Temperaturmessung während der Aushärtung ergibt einen Maximalwert von 37 °C. Die Druckfestigkeit erreicht einen Wert von 60 MPa.

### Beispiel 2:

Wie Beispiel 1, aber Verwendung eines Copolymer-Gemisches als Knochenzement der Zusammensetzung 80 % Polymethylmethacrylat/ Polymethylacrylat (94/6) + 20 % Polymethylmethacrylat/Polymethylacrylat (52/48). Erhalten wird dadurch ein standardviskoser Knochenzement, der in der genannten Abmischung zu einem gut formbaren Knochenersatz führt. Die Polymerisationszeit beträgt ca. 9 Min.. Diese Variante ist vor allem für die Applikation der plastischen Masse in den Knochen und Aushärtung in situ geeignet.

### Beispiel 3:

Aus einer Mischung von 95 g Perlpolymerisat Polymethylmethacrylat (Durchmesser 30-80 µm), 5 g Copolymer Polymethylmethacrylat/Polymethylacrylat (52/48), 25 g Hydroxylapatit-Pulver (2-5 µm) und 10 g Zirkondioxid-Pulver wird unter Zugabe von 70 ml Methylmethacrylat eine viskose Suspension hergestellt. Ein übliches Startersystem wird zugesetzt.

In einem 2-l-Becherglas wird eine 2%ige Lösung von Na-Alginat gleichmäßig gerührt und auf 50 °C erhitzt. Unter weiterem Rühren wird die Suspension tropfend in die Alginat-Lösung gegeben, so daß sich möglichst gleichmäßige Perlen ergeben. Unter den genannten Bedingungen polymerisieren die Perlen in ca. 5 Min. zu festen Partikeln, die sich nach Abstellen des Rührers am Boden absetzen. Die Partikel werden abgetrennt, gewaschen, getrocknet und klassiert.

Partikel der Fraktion 1-2 mm werden in einem kleineren Ansatz analog zu Beispiel 1 verklebt. Die Porosität beträgt in diesem Ansatz 20 %, die Druckfestigkeit 65 MPa.

### Beispiel 4:

10 g Polymethylmethacrylat/Polymethylacrylat-Copolymer (52/48) werden mit 90 g Polymethylmethacrylat-Granulat N8 (Partikelgröße ∼ 1 mm) und 0,5 g Benzoylperoxid in der Kugelmühle intensiv gemischt. 50 g dieser Mischung werden mit 10 ml eines Gemisches aus 60 Gew.-% Methylmethacrylat, 20 Gew.-% Isobornylmethacrylat und 20 Gew.-% Decylmethacrylat (enthält N,N-Dimethyl-p-toluidin) angerührt. Es entsteht sehr schnell ein zäher Teig, der nach ca. 1 Min. geknetet werden kann. Nach einer weiteren Minute Kneten ist das Material implantationsbereit. Nach Aushärtung wird ein poröses Material mit hoher Druckfestigkeit erhalten.

## Patentansprüche

1. Verfahren zur Herstellung von porösen Knochenersatzmaterialien, die ein teilweise oder vollständig interkonnektierendes Porensystem mit einem Volumenanteil von 5 bis 60 % aufweisen, dadurch gekennzeichnet, daß man
(a) 0 bis 48 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffe und Farbstoffe,
(b) 2 bis 50 Gew.% einer flüssigen Komponente, bestehend aus Acryl- und/oder Methacrylsäureestermonomeren sowie gegebenenfalls weiteren Zusätzen wie Polymerisationsbeschleunigern und Stabilisatoren, und
(c) 50 bis 98 Gew.% eines grobteiligen Granulates aus einem biokompatiblen Material mit größtem Partikeldurchmesser von 0,5 bis 10 mm
miteinander vermengt, gegebenenfalls in eine gewünschte Form bringt und dann aushärtet.

2. Verfahren nach Anspruch 1, dadurch gekennzeichnet, daß das grobteilige Granulat größte Partikeldurchmesser zwischen 1 und 5 mm, vorzugsweise um etwa 3 mm, aufweist.

3. Verfahren nach den Ansprüchen 1 oder 2, dadurch gekennzeichnet, daß das grobteilige Granulat Kugelform, angenäherte Kugelform oder Zylinderform besitzt.

4. Verfahren nach den Ansprüchen 1 bis 3, dadurch gekennzeichnet, daß das grobteilige Granulat ein ausgehärtetes Gemisch von etwa 5 bis 90 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, und von etwa 95 bis 10 Gew.% einer flüssigen Komponente, bestehend aus Acryl- und/oder Methacrylsäureestermonomeren sowie gegebenenfalls weiteren Zusätzen wie Polymerisationsbeschleunigern und Stabilisatoren, ist.

5. Verfahren nach den Ansprüchen 1 bis 4, dadurch gekennzeichnet, daß das grobteilige Granulat aus Kunststoffmaterialien auf Basis von Polyolefinen, Copolymeren von Acrylaten mit Styrol und/oder Butadion sowie Epoxidharzen besteht.

6. Verfahren nach den Ansprüchen 1 bis 5, dadurch gekennzeichnet, daß das grobteilige Granulat eine gesinterte Calciumphosphatkeramik ist.

7. Verfahren nach den Ansprüchen 1 bis 6, dadurch gekennzeichnet, daß die Feststoffkomponente und/oder das grobteilige Granulat osteoinduktive und/oder osteokonduktive Füllstoffe enthält.

8. Verfahren nach den Ansprüchen 1 bis 7, dadurch gekennzeichnet, daß die Feststoffkomponente und/oder das grobteilige Granulat pharmazeutische Wirkstoffe wie insbesondere Cytostatika, Antibiotika, Antiseptika oder knochenwachstumsfördernde Substanzen enthält.

9. Set zur Herstellung eines porösen Knochenersatzmaterials, das ein interkonnektierendes Porensystem mit einem Volumenanteil von 5 bis 60 % aufweist, zusammengesetzt aus getrennten Packungen von
(a) einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, deren Anteil etwa 0 bis 48 Gew.% des Knochenersatzmaterials beträgt,
(b) einer flüssigen Komponente, bestehend aus Acryl- und/oder Methacrylsäureestermonomeren sowie gegebenenfalls weiteren Zusätzen wie Polymerisationsbeschleunigern und Stabilisatoren, deren Anteil etwa 2 bis 50 Gew.% des Knochenersatzmaterials beträgt, und
(c) einem grobteiligen Granulat eines Polymerisates aus einem biokompatiblen Material mit größtem Partikeldurchmesser von 0,5 bis 10 mm, deren Anteil etwa 50 bis 98 Gew.% des Knochenersatzmaterials beträgt.

10. Set nach Anspruch 9, dadurch gekennzeichnet, daß die Komponenten (a) und (c) im Gemisch in einer Packungseinheit vorliegen.

11. Set nach den Ansprüchen 9 oder 10, dadurch gekennzeichnet, daß das grobteilige Granulat größte Partikeldurchmesser zwischen 1 und 5 mm, vorzugsweise um etwa 3 mm, aufweist.

12. Set nach den Ansprüchen 9 bis 11, dadurch gekennzeichnet, daß das grobteilige Granulat Kugelform, angenäherte Kugelform oder Zylinderform besitzt.

13. Set nach den Ansprüchen 9 bis 12, dadurch gekennzeichnet, daß das grobteilige Granulat ein ausgehärtetes Gemisch von etwa 5 bis 90 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, und von etwa 95 bis 10 Gew.% einer flüssigen Komponente, bestehend aus Acryl- und/oder Methacrylsäureestermonomeren sowie gegebenenfalls weiteren Zusätzen wie Polymerisationsbeschleunigern und Stabilisatoren, ist.

14. Set nach den Ansprüchen 9 bis 13, dadurch gekennzeichnet, daß das grobteilige Granulat eine gesinterte Calciumphosphatkeramik ist.

15. Set nach den Ansprüchen 9 bis 14, dadurch gekennzeichnet, daß die Feststoffkomponente und/oder das grobteilige Granulat osteoinduktive und/oder osteokonduktive Füllstoffe enthält.

16. Set nach den Ansprüchen 9 bis 15, dadurch gekennzeichnet, daß die Feststoffkomponente und/oder das grobteilige Granulat pharmazeutische Wirkstoffe wie insbesondere Cytostatika, Antibiotika, Antiseptika oder knochenwachstumsfördernde Substanzen enthält.

## Claims

1. Process for the preparation of porous bone replacement materials which have a partly or completely interconnecting pore system with a volume content of 5 to 60%, characterized in that
(a) 0 to 48% by weight of a solid component comprising a finely divided polymer of acrylic and/or methacrylic esters and, if appropriate, further additives, such as polymerization catalysts, X-ray contrast media, fillers and colourants,
(b) 2 to 50% by weight of a liquid component comprising acrylic and/or methacrylic ester monomers and, if appropriate, further additives, such as polymerization accelerators and stabilizers, and
(c) 50 to 98% by weight of coarse-particled granules of a biocompatible material having a largest particle diameter of 0.5 to 10 mm
are mixed with one another and the mixture is brought into a desired shape, if appropriate, and then hardened.

2. Process according to Claim 1, characterized in that the coarse-particled granules have a largest particle diameter of between 1 and 5 mm, preferably about 3 mm.

3. Process according to Claim 1 or 2, characterized in that the coarse-particled granules have a spherical shape, approximately spherical shape or cylindrical shape.

4. Process according to Claims 1 to 3, characterized in that the coarse-particled granules are a hardened mixture of about 5 to 90% by weight of a solid component comprising a finely divided polymer of acrylic and/or methacrylic esters and, if appropriate, further additives, such as polymerization catalysts, X-ray contrast media, fillers and colourants, and about 95 to 10% by weight of a liquid component comprising acrylic and/or methacrylic ester monomers and, if appropriate, further additives, such as polymerization accelerators and stabilizers.

5. Process according to Claims 1 to 4, characterized in that the coarse-particled granules comprise plastics materials based on polyolefins, copolymers of acrylates with styrene and/or butadion, and epoxy resins.

6. Process according to Claims 1 to 5, characterized in that the coarse-particled granules are a sintered calcium phosphate ceramic.

7. Process according to Claims 1 to 6, characterized in that the solid component and/or the coarse-particled granules comprise osteoinductive and/or osteoconductive fillers.

8. Process according to Claims 1 to 7, characterized in that the solid component and/or the coarse-particled granules comprise pharmaceutical active compounds, such as, in particular, cytostatics, antibiotics, antiseptics or bone growth-promoting substances.

9. Set for the preparation of a porous bone replacement material which has an interconnecting pore system with a volume content of 5 to 60%, composed of separate packs of
(a) a solid component comprising a finely divided polymer of acrylic and/or methacrylic esters and, if appropriate, further additives, such as polymerization catalysts, X-ray contrast media, fillers and colourants, the proportion of which is about 0 to 48% by weight of the bone replacement material,
(b) a liquid component comprising acrylic and/or methacrylic ester monomers and, if appropriate, further additives, such as polymerization accelerators and stabilizers, the proportion of which is about 2 to 50% by weight of the bone replacement material, and
(c) coarse-particled granules of a polymer of a biocompatible material with a largest particle diameter of 0.5 to 10 mm, the proportion of which is about 50 to 98% by weight of the bone replacement material.

10. Set according to Claim 9, characterized in that components (a) and (c) are present as a mixture in one pack unit.

11. Set according to Claims 9 or 10, characterized in that the coarse-particled granules have a largest particle diameter of between 1 and 5 mm, preferably about 3 mm.

12. Set according to Claims 9 to 11, characterized in that the coarse-particled granules have a spherical shape, approximately spherical shape or cylindrical shape.

13. Set according to Claims 9 to 12, characterized in that the coarse-particled granules are a hardened mixture of about 5 to 90% by weight of a solid component comprising a finely divided polymer of acrylic and/or methacrylic esters and, if appropriate, further additives, such as polymerization catalysts, X-ray contrast media, fillers and colourants, and about 95 to 10% by weight of a liquid component comprising acrylic and/or methacrylic ester monomers and, if appropriate, further additives, such as polymerization accelerators and stabilizers.

14. Set according to Claims 9 to 13, characterized in that the coarse-particled granules are a sintered calcium phosphate ceramic.

15. Set according to Claims 9 to 14, characterized in that the solid component and/or the coarse-particled granules comprise osteoinductive and/or osteoconductive fillers.

16. Set according to Claims 9 to 15, characterized in that the solid component and/or the coarse-particled granules comprise pharmaceutical active compounds, such as, in particular, cytostatics, antibiotics, antiseptics or bone growth-promoting substances.

## Revendications

1. Procédé pour préparer des matières poreuses de remplacement des os, à système poreux partiellement ou totalement interconnecté représentant une proportion en volume de 5 à 60 %, caractérisé en ce que l'on mélange entre eux :
(a) 0 à 48 % en poids d'un composant solide consistant en un polymère à l'état de fines particules d'esters acryliques et/ou méthacryliques et le cas échéant des additifs tels que des catalyseurs de polymérisation, des agents de contraste aux rayons X, des matières de charge et des colorants,
(b) 2 à 50 % en poids d'un composant liquide consistant en esters acryliques et/ou méthacryliques monomères et le cas échéant des additifs tels que des accélérateurs de polymérisation et des stabilisants, et
(c) 50 à 98 % en poids d'un granulat en particules grossières d'une matière biocompatible au diamètre de particule maximum de 0,5 à 10 mm,
le cas échéant on met le mélange sous la forme voulue puis on durcit.

2. Procédé selon la revendication 1, caractérisé en ce que le granulat à l'état de particules grossières a un diamètre de particule maximum de 1 à 5 mm et de préférence d'environ 3 mm.

3. Procédé selon les revendications 1 ou 2 caractérisé en ce que le granulat grossier est à l'état de particules sphériques, à peu près sphériques ou cylindriques.

4. Procédé selon les revendications 1 à 3, caractérisé en ce que le granulat à l'état de particules grossières est un mélange durci d'environ 5 à 90 % en poids d'un composant solide consistant en un polymère à l'état de fines particules d'esters acryliques et/ou méthacryliques et le cas échéant en additifs tels que des catalyseurs de polymérisation, des agents de contraste aux rayons X, des matières de charge et des colorants, et d'environ 95 à 10 % en poids d'un composant liquide consistant en esters acryliques et/ou méthacryliques monomères et le cas échéant des additifs tels que des accélérateurs de polymérisation et des stabilisants.

5. Procédé selon les revendications 1 à 4, caractérisé en ce que le granulat à l'état de particules grossières consiste en résines synthétiques à base de polyoléfines, de copolymères d'acrylates et de styrène et/ou de butadiène et de résines époxydiques.

6. Procédé selon les revendications 1 à 5, caractérisé en ce que le granulat à l'état de particules grossières consiste en une céramique frittée de phosphate de calcium.

7. Procédé selon les revendications 1 à 6, caractérisé en ce que le composant solide et/ou le granulat grossier contiennent des matières de charge ostéo-inductrices et/ou ostéo-conductrices.

8. Procédé selon les revendications 1 à 7, caractérisé en ce que le composant solide et/ou le granulat grossier contiennent des substances actives pharmaceutiques, en particulier des agents cytostatiques, des antibiotiques, des antiseptiques ou des substances favorisant la croissance des os.

9. Nécessaire pour la préparation d'une matière poreuse de remplacement des os à système poreux interconnecté représentant une proportion en volume de 5 à 60 %, qui se compose des emballages séparés de
(a) un composant solide consistant en un polymère en fines particules d'esters acryliques et/ou méthacryliques et le cas échéant des additifs tels que des catalyseurs de polymérisation, des agents de contraste aux rayons X, des matières de charge et des colorants, dont la proportion représente d'environ 0 à 48 % du poids de la matière de remplacement des os,
(b) un composant liquide consistant en esters acryliques et/ou méthacryliques monomères et le cas échéant des additifs tels que des accélérateurs de polymérisation et stabilisants, dont la proportion représente d'environ 2 à 50 % du poids de la matière de remplacement des os, et
(c) un granulat en particules grossières d'un polymère consistant en une matière biocompatible au diamètre de particule maximum de 0,5 à 10 mm, dont la proportion représente d'environ 50 à 98 % du poids de la matière de remplacement des os.

10. Nécessaire selon la revendication 9, caractérisé en ce que les composants (a) et (c) sont à l'état de mélange dans un seul emballage.

11. Nécessaire selon les revendications 9 ou 10, caractérisé en ce que le granulat grossier a un diamètre de particule maximum de 1 à 5 mm, de préférence d'environ 3 mm.

12. Nécessaire selon les revendications 9 à 11, caractérisé en ce que le granulat grossier est à l'état de particules sphériques, à peu près sphériques ou cylindriques.

13. Nécessaire selon les revendications 9 à 12, caractérisé en ce que le granulat grossier consiste en un mélange durci d'environ 5 à 90 % en poids d'un composant solide consistant lui-même en un polymère en fines particules d'esters acryliques et/ou méthacryliques et le cas échéant des additifs tels que des catalyseurs de polymérisation, des agents de contraste aux rayons X, des matières de charge et des colorants, et d'environ 95 à 10 % en poids d'un composant liquide consistant en esters acryliques et/ou méthacryliques monomères et le cas échéant des additifs tels que des accélérateurs de polymérisation et des stabilisants.

14. Nécessaire selon les revendications 9 à 13, caractérisé en ce que le granulat en particules grossières consiste en une céramique frittée de phosphate de calcium.

15. Nécessaire selon les revendications 9 à 14, caractérisé en ce que le composant solide et/ou le granulat grossier contiennent des matières de charge ostéo-inductrices et/ou ostéo-conductrices.

16. Nécessaire selon les revendications 9 à 15, caractérisé en ce que le composant solide et/ou le granulat grossier contiennent des substances actives pharmaceutiques, en particulier des agents phytostatiques, des antibiotiques, des antispetiques ou des substances favorisant la croissance des os.
